# EUROPÄISCHE PATENTSCHRIFT

(11) **EP 2 531 178 B1**
(45) Veröffentlichungstag und Bekanntmachung des Hinweises auf die Patenterteilung: **04.06.2014**
(21) Anmeldenummer: 11700067.9
(22) Anmeldetag: 07.01.2011
(51) Int. Cl.: A61K 9/20, C01F 5/24

(54) **DIREKT VERPRESSBARES MAGNESIUMHYDROXIDCARBONAT**
DIRECTLY COMPRESSIBLE MAGNESIUM HYDROXIDE CARBONATE
CARBONATE D'HYDROXYDE DE MAGNÉSIUM COMPRESSIBLE PAR VOIE DIRECT

(30) Priorität: 03.02.2010 EP 10001099
(43) Veröffentlichungstag der Anmeldung: 12.12.2012
(73) Patentinhaber: Merck Patent GmbH, 64293 Darmstadt (DE)
(72) Erfinder: DOHMEN, Marianne, 86565 Gachenbach (DE); FIEDLER, Ralf, 64743 Beerfelden (DE); MUELLER, Heike, 64291 Darmstadt (DE); KLATYK, Jens, 64347 Griesheim (DE); PETH, Hans-Kurt, 64665 Alsbach-Haehnlein (DE); MODDELMOG, Guenter, 64354 Reinheim (DE); WEDEL, Thorsten, 64589 Stockstadt/Rhein (DE)
(86) Internationale Anmeldenummer: PCT/EP2011/000045
(87) Internationale Veröffentlichungsnummer: WO 2011/095269

(56) Entgegenhaltungen:
- EP-A2- 0 460 923
- JP-A- 60 054 915
- US-A- 5 240 692

## Beschreibung

Die vorliegende Erfindung betrifft ein direkt verpressbares Magnesiumhydroxidcarbonat und ein Verfahren zu dessen Herstellung, sowie dessen Verwendung.

### Stand der Technik

Basisches Magnesiumcarbonat bzw. Magnesiumhydroxidcarbonat, mit der chemischen Zusammensetzung : 4MgCO3×Mg(OH)2×5H2O kann aus Magnesiumcarbonat (MgCO₃) hergestellt werden. Magnesiumcarbonat ist ein weißes, in Wasser sehr schwer lösliches Pulver. Es entsteht aus wässriger Lösung nur, wenn diese viel überschüssige Kohlensäure enthält. Magnesiumcarbonat kann mit 5, 3 und 1 Mol Kristallwasser kristallisieren und wird beim Kochen mit Wasser allmählich zu dem basischen Magnesiumcarbonat zersetzt. Entsprechende Verfahren zur Herstellung sind seit langem bekannt.

Zur Herstellung von pharmazeutischen Zusammensetzungen und für technische Anwendungen wird viel häufiger basisches Magnesiumcarbonat (Magnesia alba) als das reine Magnesiumcarbonat verwendet. Üblicherweise wird Magnesiumhydroxidcarbonat durch Ausfällen aus einer Magnesiumsulfatlösung mit Soda erhalten.

Das Magnesiumhydroxidcarbonat ist ein schneeweißes, sehr leichtes, lockeres, wasserunlösliches Pulver, das sich in Säuren viel schneller auflöst als Magnesit. Es dient daher im Allgemeinen als Ausgangsmaterial für die Herstellung anderer Magnesium-Verbindungen.

Ferner verwendet man Magnesiumhydroxidcarbonat in Antazida gegen Magenübersäuerung, als Zahnpulver, Wundstreupulver, als Gegenmittel bei Vergiftungen mit Säuren, Arsenik und Metallsalzen, zur Herstellung von Pudern, Putzpulvern usw. Es wird aber auch als Füllmittel für Farben, Papier, Kautschuk, als feuerfestes Material, zur Hitzeisolierung u. dgl. verwendet. Insbesondere wird Magnesiumhydroxidcarbonat häufig als Mineralstoff zur Magnesiumanreichung von Lebensmitteln, Pharmazeutika und diätetischen Zubereitungen verwendet. Es wird bevorzugt auch als Bestandteil in Komprimaten/Tabletten wie z.B. in Kau- und Brausetabletten eingesetzt, weil es einen verhältnismäßig hohen Magnesiumgehalt aufweist und zu einem günstigen Preis erhältlich ist. In Brausetabletten dient es darüber hinaus als Kohlendioxidquelle zur Erzeugung des Brauseeffekts. Solche Komprimate werden bevorzugt durch Direkttablettierungsprozesse, d.h. ohne vorherige Granulationsschritte, hergestellt.

Bislang bekanntes pulverförmiges Magnesiumhydroxidcarbonat kann jedoch wegen seiner schlechten Fließeigenschaften und wegen fehlender Kompressibilität nicht ohne spezielle Zusätze oder spezielle Vorbehandlung direkt tablettiert werden.

In EP 0 460 923 ist die Herstellung eines basischen Magnesiumcarbonats beschrieben, für das BET-Oberflächen von 10-70m²/g beansprucht werden. Die offenbarten Beispiele weisen BET-Oberfläche bis max. 38 m²/g (Tabelle 1) auf. Für diese Materialien wird eine mittlere Teilchengröße von 1 bis 50 µm beansprucht, während die durch Beispiele belegte durchschnittliche Korngröße nur im Bereich 3,8 bis 6,0 µm liegt (Tabelle 1). Im Handel sind tatsächlich jedoch nur entsprechende Produkte von der Fa. Lehmann & Voss "PharMagnesia MC Type A Granulat" erhältlich, welche eine BET-Oberfläche von 32 m²/g aufweisen.

Die Herstellung des basischen Magnesiumcarbonats erfolgt in EP 0 460 923 durch eine Reaktion aus Magnesiumsulfat Heptahydrat mit Natriumcarbonat unter Zusatz eines "Crystallizing Assistant" (Tabelle 1). Dabei ist es zur Erzielung einer hohen BET-Oberfläche und eines hohen Porenvolumens unbedingt notwendig die Reaktionspartner als feststoffe in das Reaktionsgefäß einzubringen, da sonst kein erfindungsgemäßes Produkt zu erhalten ist. Der Einsatz bereits vorgelöster Reaktionsprodukte wird explizit ausgeschlossen.

Untersucht man die Verpressungseigenschaften der auf dem Markt befindlichen und als "direktverpreßbar" ausgelobten reinen Magnesiumhydroxidcarbonate, so sind diese nicht überzeugend. Bessere Tablettierungseigenschaften werden durch die Kombination des spröden Magnesiumsalzes mit einer plastisch wirkenden Komponente, beispielsweise in Form von Stärke, erzielt. Solche Zusammensetzungen sind zwar besser verpressbar, haben aber aufgrund des Bindmittelzusatzes einen verringerten Magnesiumgehalt. Darüber hinaus sind sie nicht mehr durch eine einfache Arzneibuchmonographie charakterisierbar. Insbesondere Letzteres kann zu Registrierungs- bzw. Dokumentationsproblemen für die Anwender in der Herstellung von pharmazeutischen Formulierungen führen. Außerdem ist die Herstellung dieser Zusammensetzungen, bedingt durch zusätzliche Prozessschritte, wie z.B. einer erforderlichen Granulation, recht aufwändig.

### Aufgabenstellung

Aufgabe der vorliegenden Erfindung ist es daher, ein preiswertes, einfach herstellbares Magnesiumhydroxidcarbonat zur Verfügung zu stellen, welches einen möglichst hohen Magnesiumgehalt aufweist und den Reinheitsanforderungen der üblichen Pharmakopöen genügt und welches ohne weitere Zusätze in Komprimationsprozessen direkt einsetzbar ist und verpreßt werden kann. Weiterhin ist es Aufgabe der vorliegenden Erfindung, ein einfach durchführbares, preiswertes Verfahren zur Herstellung eines solchen direkt verpreßbaren Magnesiumhydroxidcarbonats zur Verfügung zu stellen.

### Lösung der Aufgabe

Die Aufgabe der vorliegenden Erfindung wird dadurch gelöst, dass in einem kontinuierlichen Verfahren Magnesiumhydroxidcarbonat aus wässrigen Lösungen der Ausgangssalze ausgefällt wird, und anschließend einem Trocknungsprozess unterzogen wird. Durch das geänderte Herstellungsverfahren wird ein Magnesiumhydroxidcarbonat mit veränderter Erscheinungsform erhalten, welches besonders vorteilhafte galenische Eigenschaften aufweist und im Vergleich zu handelsüblichen Produkten eine ungewöhnlich hohe BET-Oberfläche von mindestens 44 bis 70 m²/g, vorzugsweise größer 50 m²/g, eine Schüttdichte von 0,40 - 0,60 g/ml und eine Stampfdichte von 0,50 - 0,80 g/ml aufweist. Für die Begriffe Schüttdichte und Stampfdichte sind auch die Bezeichnungen Schüttgewicht und Stampfgewicht gebräuchlich. Dieses neue erfindungsgemäße Material ist sehr gut direkt-tablettierbar und entspricht in seinen Reinheitskriterien den Anforderungen der PhEur, BP, USP und E 504 für sogenanntes Magnesiumhydroxidcarbonat schwer (schweres, basisches Magnesiumcarbonat). Weiterhin weist es vorzugsweise mittlere Partikeldurchmesser (Laser; D_{0,50}) im Bereich von 20 und 60 µm, insbesondere im Bereich von 24 bis 60 µm auf.

Insbesondere erfolgt die Lösung der vorliegenden Aufgabe durch zur Verfügungstellen von direkt verpressbarem Magnesiumhydroxidcarbonat, (schwer, gemäß den Reinheitsanforderungen der PhEur, BP, USP und E 504), welches durch Kompression mit einer Preßkraft von im Bereich von 10 kN bis 20 kN zu Tabletten mit Härten im Bereich >80 N bis >200 N und einer Friabilität < 0,2 Gew.-%direkt verpreßbar ist. Dieses Magnesiumhydroxidcarbonat läßt sich ebenfalls durch Kompression mit einer Preßkraft von im Bereich von 10 kN bis 20 kN zu Tabletten mit einer Friabilität <0,1 Gew.-% verpressen.

Erfindungsgemäß ist dieses Magnesiumhydroxidcarbonat in einer kontinuierlichen Reaktion herstellbar, wobei in einem Rohrreaktor eine erwärmte Lösung eines Magnesiumsalzes, worin der Magnesiumgehalt in der Lösung 2 - 11 Gew.-%, insbesondere bevorzugt 3 - 6 Gew.-%, beträgt, und eine erwärmte Lösung eines Alkali-oder Erdalkalicarbonats, worin der Carbonatgehalt in der Lösung 2 - 18 Gew.-%, bevorzugt 3 - 15 Gew.-%, beträgt, wobei sich eine Temperatur im Bereich von 60 bis 70°C und ein pH-Wert im Bereich von 8,5 - 9,0 einstellt, vermischt werden . Anschließend erfolgt die Abfiltration des ausgefällten Magnesiumhydroxidcarbonats und eine nachfolgende Trocknung in einem Konvektionstrockner auf einen Gehalt von 40 bis 43,5 Gew.-% (berechnet als MgO).

Gegenstand der vorliegenden Erfindung ist auch die Verwendung des direkt verpreßbaren Magnesiumhydroxidcarbonats gemäß einem oder mehreren der Ansprüche 1 bis 5 als Bestandteil von Tablettenformulierungen, gemäß der Ansprüche 12 bis 16, sowie in Tabletten mit den Eigenschaften gemäß Anspruch 6.

Insbesondere ist ein Verfahren zur Verstellung des erfindungsgemäßen, direkt verpressbaren Magnesiumhydroxidcarbonats Gegenstand der vorliegenden Erfindung. Dieses Verfahren ist dadurch gekennzeichnet, dass
a) eine erwärmte Lösung eines Magnesiumsalzes, worin der Magnesiumgehalt 2 - 11 Gew.-%, bevorzugt 3 - 10 Gew.-%, insbesondere 3 - 6 Gew.-%, beträgt, und eine erwärmte Lösung eines Alkali-oder Erdalkalicarbonats, worin der Carbonatgehalt 2 - 18 Gew.-%, bevorzugt 3 - 16 Gew.-%, besonders bevorzugt 3 - 15 Gew.-% und insbesondere bevorzugt 7 - 10 Gew.-%, beträgt, kontinuierlich in einen Rohrreaktor gepumpt und vermischt werden, so dass sich in der Reaktionslösung eine Temperatur von 60 bis 70°C und ein pH-Wert im Bereich von 8,5 - 9,0 einstellt und das gebildete Magnesiumhydroxidcarbonat ausfällt und
b) aus der produkthaltigen Reaktionsmischung, gegebenenfalls nach einiger Zeit ruhen lassen, das ausgefällte Produkt abfiltriert und getrocknet wird. Vorzugsweise erfolgt die nachfolgende Trocknung in einem Konvektionstrockner, bevorzugt einem Wirbelschichttrockner. Hierbei wird das abfiltrierte Produkt auf einen Gehalt von 40 bis 43,5 Gew.-% (berechnet als MgO) getrocknet. Vor der Abtrennung des Produkts ist es vorteilhaft, die produkthaltige Reaktionsmischung bei einer Temperatur im Bereich von 55 - 65 °C für eine Zeit von 10 - 45 Minuten ruhen zu lassen.

### Detaillierte Beschreibung der Erfindung

Durch das erfindungsgemäße Magnesiumhydroxidcarbonat erhält der Galeniker ein hinsichtlich der Direkttablettierungseigenschaften optimiertes Produkt, wodurch Tabletten oder Komprimate mit hochdoslertem Magnesiumgehalt preiswert in einfacher Weise herstellbar sind. Insbesondere ist dieses Magnesiumhydroxidcarbonat ohne Vorbehandlung in einem besonderen Granulierungsschritt direkt zur Tablettierung einsetzbar.

Zur Herstellung des erfindungsgemäßen Produkts werden an sich ein Alkali- oder Erdalkalicarbonat sowie ein lösliches Magnesiumsalz, beispielsweise ein Chlorid, Sulfat oder ähnliches lösliches Salz in Form von erwärmten Lösungen, die getrennt in einen geeigneten Reaktor gepumpt werden in einem kontinuierlichen Prozess zur Umsetzung gebracht. Das entstehende Produkt fällt aus und wird, gegebenenfalls nach zusätzlichen Behandlungsschritten, über einen Trocknungsprozess, beispielsweise durch eine Konvektionstrocknung, bevorzugt durch Trocknung in der Wirbelschicht, in eine pulvrige Form überführt. Darüber hinaus ist keine weitere Behandlung notwendig. Das so erhaltene Material entspricht den Reinheitskriterien der Lebensmittel- und Pharma-Industrie und zeichnet sich durch hohe BET-Oberflächen im Bereich von 44 - 70 m²/g aus. Die Direkttablettierung kann einfach durch die Abmischung mit einem in der pharmazeutischen Industrie üblichen Tablettierhilfsmittel und anschließende Verpressung erfolgen. Durch Verwendung des so hergestellten Magnesiumhydroxidcarbonats können bei gleichen Presskräften höhere Tablettenhärten erhalten werden als bei Verwendung bisher kommerziell erhältlicher direkt verpreßbarer Magnesiumhydroxidcarbonate.

Die Herstellung der erfindungsgemäßen Produkte erfolgt vorzugsweise in einem kontinuierlichen Verfahren. Zur Durchführung dieses Verfahrens werden die Edukte in Wasser gelöst. Bei den Edukten handelt es sich um Carbonate, insbesondere Alkali- oder Erdalkalicarbonate und geeignete Magnesiumsalze, wie Chlorid, Sulfat oder ähnliche lösliche Salze, insbesondere bevorzugt um Magnesiumchlorid.

Unter Erwärmen wird aus den Carbonaten eine wässrige Lösung hergestellt, worin der jeweilige Carbonatanteil in einer Konzentration von etwa 2 - 18 Gew.-%, bevorzugt etwa 3 - 15 Gew.-%, besonders bevorzugt 7 - 10 Gew.- %, enthalten ist. Aus dem Magnesiumsalz, bevorzugt Magnesiumchlorid, wird eine wässrige Lösung hergestellt mit einem Magnesiumgehalt im Bereich von 2 - 11 Gew.-%, bevorzugt von 3 - 10 Gew.-%, insbesondere bevorzugt 3 - 6 Gew.-%. Die Gehaltseinstellungen der Lösungen erfolgen praktischerweise über eine Korrelation zur Dichte der Lösungen, wobei die Dichte auf verschiedene, dem Fachmann bekannte Methoden bestimmt werden kann. Für die industrielle Anwendung sind elektroakustische Methoden, z. B. mittels Densimeter mit Schwingungswandler, vorzuziehen, da sie einfach durchzuführen und direkt online auswertbar sind. Die Herstellung der Ausgangslösungen lässt sich auf diese Weise in Verbindung mit geeigneten Dosiervorrichtungen in einfacher Weise automatisieren. Je nach eingesetztem Magnesiumsalz löst es sich in Wasser in einer exothermen Reaktion. Sofern es erforderlich ist, wird die erhaltene Magnesiumsalzlösung erwärmt. Um eine schnelle Reaktion zu gewährleisten, wird in der Magnesiumsalzlösung der pH-Wert in einem Bereich zwischen 4.5 und 6.0, vorzugsweise in einem Bereich zwischen 5 - 5.5 eingestellt. Bei Bedarf kann durch Zugabe der komplementären Säure oder einer geeigneten Base, wie MgO der pH-Wert einjustiert werden.

Die erhaltenen Lösungen werden anschließend unter kontrollierten Bedingungen kontinuierlich miteinander vermischt. Dazu werden die Lösungen erwärmt und unter Einhaltung der Temperatur in einem nahezu konstanten Bereich miteinander vermischt. Gleichzeitig wird der pH-Wert in einem bestimmten Bereich eingestellt und kontrolliert. Ohne Zusatz eines "Kristallisationshilfsmittels" wird auf diese Weise ein reines Produkt erhalten, das den Anforderungen der Pharmakopöen entspricht, und direkt verpreßbar ist.

Erfindungsgemäß kann diese Fällungsreaktion in jedem Reaktionsgefäß durchgeführt werden, das zur Durchführung von kontinuierlichen Reaktionen in flüssiger Phase geeignet ist und in dem eine zuverlässige Durchmischung der zugeführten Medien erfolgen kann. Als besonders geeignet für die Herstellung des erfindungsgemäßen Magnesiumhydroxydcarbonats hat sich aber die Verwendung von Rohrreaktoren erwiesen, in die die vorgewärmten Ausgangslösungen kontinuierlich gepumpt werden, und aus denen , je nach Pumpgeschwindigkeit, nach einer sich in Folge einstellenden mittleren Verweilzeit das entstehende produkthaltige Reaktionsgemisch laufend abfließt. Als besonders geeignet haben sich in den durchgeführten Versuchen mittlere Verweilzeiten im Reaktor von 4 bis 20 min, insbesondere von 7 - 15 min erwiesen.

In dem so erhaltenen Reaktionsgemisch ist bereits durch Reaktion der Salze entstandenes, ausgefälltes, kristallines Produkt enthalten, das an sich direkt durch bekannte Methoden abgetrennt und getrocknet werden kann.

Für die Durchführung der kontinuierlichen Reaktion hat sich insbesondere die Verwendung eines Rohrreaktors mit einem Innendurchmesser von 300 mm und einer Länge von 3300 mm bewährt. Die Dimensionen eines solchen geeigneten Rohrreaktors können jedoch entsprechend den gewünschten Durchsätzen verändert werden, sofern eine geeignete Durchmischung der Reaktionsflüssigkeiten erhalten bleibt. Entsprechende Veränderungen sind dem Fachmann, der sich mit dem scale-up, bzw. scale-down chemischer Reaktionsapparaturen auskennt durch geeignete Versuche ohne weiteres möglich.

Zur Durchführung der Reaktion in dem hier bevorzugten Rohrreaktor werden die separat erwärmten Salzlösungen mittels Pumpen derart in den Rohreaktor eingeführt, dass der Rohreaktor mit einer Flüssigkeitsmenge von etwa 1300 l/h, vorzugsweise etwa 1500 l/h, durchflossen wird.

Die Fällung des erwünschten Magnesiumhydroxidcarbonats erfolgt bei Einhaltung eines pH-Werts in der Reaktionslösung im Bereich von 8,5 bis 9,0 und einer Temperatur im Bereich von 60 - 70 °C. An sich stellt sich der pH-Wert beim Vermischen der Ausgangslösungen automatisch auf den gewünschten Wert ein. Bei Bedarf lässt sich der pH-Wert durch Zugabe von geringen Mengen Magnesiumoxid oder einer korrespondierenden Säure einstellen. Dieses kann auch noch während der Reaktion erfolgen, wenn der Reaktor mit entsprechenden Meß- und Dosierungseinrichtungen ausgestattet ist. Günstiger ist es jedoch, wenn vorab der pH-Wert der Ausgangslösungen so eingestellt ist, dass sich der pH-Wert automatisch beim Vermischen der Ausgangslösungen in dem gewünschten Bereich einstellt.

Durch Versuchsreihen wurde gefunden, dass verbesserte Produkteigenschaften erzielt werden können, wenn das erhaltene, produkthaltige Reaktionsgemisch in geeigneten Vorratsgefäßen gesammelt wird, und man es für einige Zeit ruhen lässt. Auf diese Weise kann sowohl die Reaktion der Salze als auch die Fällung des gewünschten Produkts vervollständigt werden. Aus dem Reaktionsgemisch kann direkt oder nach einer entsprechenden Ruhezeit ausgefälltes, kristallines Produkt abgetrennt werden. Es hat sich an sich als vorteilhaft erwiesen, die durch die Reaktion erhaltenen Suspensionen zu sammeln und ruhen zu lassen. Dieses hat auch den Vorteil, dass die erhaltene Suspension gleichmäßig der Filtrationsanlage zugeführt werden kann.

Die Produktabtrennung kann in einer dem Fachmann bekannten Weise erfolgen, beispielsweise durch Zentrifugieren oder Filtration. Besonders geeignet ist die Abtrennung durch Filtration. Zur kontinuierlichen Produktabtrennung hat sich die Bandfiltration als geeignet erwiesen.

Dabei wird die zu filtrierende Flüssigkeit kontinuierlich auf eine Filterbahn geleitet, die aus einem geeigneten Vlies oder Gewebe besteht. Die abzutrennenden Partikel werden auf der Filterbahn zurückgehalten und die von Partikeln befreite Flüssigkeit wird zur Entsorgung abgeleitet. Der auf der Filterbahn zurückbleibende Rückstand bildet einen sogenannten Filterkuchen, der noch auf dem Filterband zur Reinigung mit reinem Wasser, aber auch mit geeigneten Lösungsmitteln gewaschen werden kann. Direkt im Anschluss an die Filtration wird der gewaschene Filterkuchen der Trocknung zugeführt. Der ganze Vorgang kann kontinuierlich und vollautomatisch erfolgen, ohne dass der Flüssigkeitsstrom unterbrochen werden muß.

Der sich an die Abtrennung des ausgefällten Magnesiumhydroxidcarbonts anschließende Trocknungsschritt kann auf verschiedene, dem Fachmann bekannte Weisen erfolgen. Um ein Verbacken des erhaltenen Produkts zu vermeiden, erfolgt die umgehende Trocknung bevorzugt durch eine Konvektionstrocknung. Besonders bevorzugt erfolgt die Trocknung in der Wirbelschicht. Zu diesem Zweck wird das abgetrennte feuchte Magnesiumhydroxidcarbonat in die Wirbelschicht des entsprechenden Trockners überführt und getrocknet. Die in den Trockner eingeblasene Luft kann dabei eine Temperatur von < 250°C aufweisen. Vorzugsweise erfolgt jedoch die Trocknung bei moderateren Temperaturen. Das feuchte Magnesiumhydroxidcarbonat wird auf diese Weise auf einen Gehalt von 40 bis 43,5 Gew.-% (berechnet als MgO) getrocknet.

Vorzugsweise wird zur Trocknung ein geeigneter Konvektionstrockner bzw. Wirbelschichttrockner eingesetzt, der mit erwärmter Luft betrieben wird, die bevorzugt eine Temperatur im Bereich von 70 bis 140°C aufweist. Beispielsweise ist für diesen Zweck ein Wirbelschichttrockner vom Typ WST/WSG der Firma Glatt (Deutschland) geeignet. Es kann aber auch ein vergleichbares im Handel erhältliches Gerät eingesetzt werden.

Zur Trocknung werden in einem Wirbelbettgerät [GPCG 5/Fa.Glatt (Deutschland)] eine bestimmte Menge abfiltriertes Produkt vorgelegt. Das Material wird mit Warmluft durchströmt bis es sich in seine Feinanteile zerlegt. Wird dabei die Zuluft mit einer Temperatur in einem Bereich von etwa 70° bis <250°C zugeführt, vorzugsweise im Bereich von 70 bis 140°C, stellt sich die Temperatur der Abluft bei einer bestimmten durchströmenden Luftmenge in einem entsprechend niedrigeren Bereich ein. Vorzugsweise wird die durchströmende Luftmenge auf etwa 370 bis 450 Nm³/h einjustiert. Dabei stellt sich die Ablufttemperatur auf etwa 35 bis 65°C ein, wenn die Temperatur der Zuluft im bevorzugten Bereich liegt. Üblicherweise beginnt das Material nach etwa 15 bis 20 Minuten Trocknung zu fluidisieren. Zu diesem Zeitpunkt kann die Abluftmenge auf 135 bis 165 Nm³/h reduziert werden unter Beibehaltung des Temperaturprofils. Die Trocknung wird solange fortgeführt, bis das Produkt die gewünschte Feuchte aufweist. Eventuell im Produkt enthaltene hartnäckige Verklumpungen können dabei durch eine Siebung über ein geeignetes Sieb beseitigt werden. Zu diesem Zweck kann ein Sieb mit einer Maschenweite von 710µm oder feiner eingesetzt werden. Der Trocknungsprozess wird beendet und eine Gehaltsbestimmung gem. Ph.Eur. durchgeführt. Sollte die Gehaltsbestimmung (berechnet als MgO) zu niedrige Werte zeigen, muss nachgetrocknet werden.

Auf die beschriebene Weise wird das gemäß Ph.Eur., USP, BP, E 504 als "Magnesiumcarbonat basisch schwer" charakterisierte Magnesiumhydroxidcarbonat mit verbesserten Eigenschaften bzgl. der Tablettierungseigenschaften erhalten.

Das erhaltene Produkt weist auch ohne den Zusatz eines Bindemittels bzw. ohne vorherige zusätzliche Granulierung sehr gute Tablettierungseigenschaften auf, d.h. das an sich spröde Magnesiumhydroxidcarbonat lässt sich schon bei geringen Preßkräften zu Tabletten mit guten Härten verarbeiten, die wiederum einen wesentlich geringeren Abrieb als herkömmliche vergleichbare Tabletten aufweisen. Das erfindungsgemäße Magnesiumhydroxidcarbonat verhält sich somit deutlich besser als vergleichbare im Handel erhältliche DC-Magnesiumcarbonate.

Selbst im Vergleich mit den im Handel erhältlichen Qualitäten, bei denen es sich um durch Zusatz eines Binders (10% Stärke) in eine direktverpreßbare Form überführte Magnesiumcarbonate handelt, sind die Tablettierungseigenschaften des erfindungsgemäßen Materials wenigstens gleichwertig oder besser.

Darüber hinaus wurde durch Versuche gefunden, dass das erfindungsgemäße Material neben einer deutlich größeren BET-Oberfläche auch ein deutlich vergrößertes BET-Porenvolumen aufweist als im Handel erhältliche Produkte. Diese veränderten Eigenschaften sind äußerlich verbunden mit einer veränderten Korngröße und Kornstruktur und in der Verwendung zur Herstellung von Tabletten mit einer verbesserten Kompressibilität. Das erhaltene Magnesiumhydroxidcarbonat, welches den Anforderungen der PhEur, BP, USP ung E 504 entspricht, lässt sich in einfacher Weise ohne weitere Tablettierhilfsmittel direkt zu Tabletten verpressen und führt bei einer Kompression mit einer Presskraft von 10 kN bis 20 kN zu Tabletten mit Härten im Bereich von >80 N bis > 200N bei gleichzeitiger Friabilität von < 0,2 Gew.-%, insbesondere < 0,1 Gew.-%.

Im Einzelnen besitzt dieses verbesserte Material vergrößerte BET-Oberflächen im Bereich von 44 bis 70 m²/g, vorzugsweise größer 50 m²/g. Aber auch das BET-Porenvolumen dieses Produkts ist gegenüber im Handel erhältlichem DC-Magnesiumcarbonat deutlich erhöht. Das so charakterisierte Magnesiumhydroxidcarbonat besitzt dabei eine Schüttdichte im Bereich von 0,25 - 0,80 g/ml, insbesondere im Bereich von 0,40 bis 0,60 g/ml, und eine Stampfdichte im Bereich von 0,35 - 0,90 g/ml, insbesondere im Bereich von 0,50 bis 0,80 g/ml.

Dagegen ist jedoch die Kornstruktur deutlich gröber als bei entsprechenden im Handel erhältlichen Produkten: die D(_{0.50}) Werte der gemessenen mittlere Teilchengrößen liegen im Bereich von 20 bis 60 µm, insbesondere im Bereich von 24 bis 60 µm.

Das erfindungsgemäß hergestellte, direkt verpreßbare Magnesiumhydroxidcarbonat kann aufgrund seiner verbesserten Eigenschaften verwendet werden als Bestandteil in wirkstoffhaltigen Tablettenformulierungen, Kau- und Lutschtabletten, Brausetabletten, Brausepulvern, in Kapselformulierungen oder in Pulverzubereitungen zur Magnesiumanreichung. Es ist aber auch hervorragend geeignet zur Herstellung von Tablettenformulierungen, welche Vitamine, Mineralstoffe, Spurenelemente, funktionelle Lebensmittelbestandteile enthalten oder zur Herstellung von Wirkstoffen enthaltenden Tablettenformulierungen, oder von Tablettenformulierungen, welche synthetische oder natürliche Farbstoffe, natürliche und/oder naturidentischen Aromastoffen und/oder andere geschmacksgebende Substanzen wie z.B. aus der Gruppe Aspartam, Saccharin, Acesulfam K, Neohesperidin, Sucralose, Thaumatin und Steviosid, oder Fruchtaromen, Fruchtsäuren, geschmacksgebende Pflanzenauszüge und pharmazeutische oder diätetische Wirkstoffe enthalten.

### Methoden

Im Folgenden werden die verwendeten Geräte und Verfahren zur Charakterisierung der Stoffeigenschaften wiedergegeben:
1. Die Schüttdichte wird gemäß DIN EN ISO 60: 1999 (Deutsche Fassung) bestimmt. Die Angabe in der vorliegenden Beschreibung, den Beispielen und den Tabellen erfolgt in "g/ml"
2. Die Stampfdichte der erhaltenen Produkte wird gemäß DIN EN ISO 787-11: 1995 (Deutsche Fassung) bestimmt. Die Angabe in der vorliegenden Beschreibung, den Beispielen und den Tabellen erfolgt in "g/ml"
3. Der Schüttwinkel der erhaltenen Produkte wird gemäß DIN ISO 4324 (Deutsche Fassung) bestimmt. Die Angabe in der vorliegenden Beschreibung, den Beispielen und den Tabellen erfolgt als "Grad"
4. Die Tablettierungsprüfung erfolgt folgendermaßen:
   492,5g des auf seine Tablettierungseigenschaften zu prüfende Material werden mit 7,5g Parteck LUB MST (Magnesiumstearat pflanzlich) EMPROVE exp PhEur, BP, JP, NF, FCC Art.No. 1.00663 (Merck KGaA, Deutschland) versetzt; das Magnesiumstearat wird zuvor über ein 250µm Sieb abgelegt, und 5 Minuten in einem verschlossenen Edelstahlbehälter (Fassungsvolumen: ca. 2 l, Höhe: ca. 19,5 cm, Durchmesser: ca. 12 cm; Außenmaße) auf einem Labor-Taumelmischer (Turbula, Fa. Willy A. Bachofen, Schweiz) gemischt. Die Verpressung zu 500 mg Tabletten (11 mm Stempel, rund, flach, mit Facette) erfolgt auf einer instrumentierten Excenter-Tablettiermaschine Korsch EK 0-DMS (Fa. Korsch, Deutschland) mit dem Auswertesystem Catman 5.0, Fa. Hottinger Baldwin Messtechnik - HBM (Deutschland).
   Je getesteter Preßkraft (Soll-Einstellungen:5+/-1, 10+1-2, 20+/-2 und 30+/- 2 kN; die effektiv gemessenen Ist-Werte sind in den Beispielen angegeben) werden mindestens 100 Tabletten zur Auswertung der Preßdaten und deren galenischen Kennzahlen hergestellt.
5. Bestimmung der Tablettenhärte, Durchmesser und Höhen: Erweka TBH 30 MD; Fa. Erweka (Deutschland); Durchschnittsdaten (arithmetische Mittelwerte) aus jeweils 20 Tablettenmessungen pro Preßkraft
6. Bestimmung des Tablettenabriebs: Friabilitätsprüfgerät Fa. Erweka (Deutschland); Geräteparameter und Ausführung der Messungen gemäß Ph.Eur. 6. Ausgabe "Friabilität von nicht überzogenen Tabletten"
7. Tablettenmasse: Durchschnittswert (arithmetischer Mittelwerte) aus der Wägung von 20 Tabletten je Preßkraft; Waage: Mettler AT 201, Fa. Mettler (Deutschland)
8. Bestimmung des Trockenverlusts
   gemäß Ph.Eur. 6. Ausgabe, Grundwerk 2008, Band 1, Seite 70, Methode 2.2.32 d) bei 130°C
9. Bestimmung der Partikelgrößenverteilung:
   Laserstreuung mit Naßdispergierung Mastersizer 2000 Ver. 5.22, Serial Number: 34403-97 mit Dispergiereinheit Hydro 2000S (A) von Malvem Instruments Ltd. (UK); Dispergiermittel: vollentsalztes Wasser, RI 1.330; Pump Speed: 2000 rpm; Stirrer Speed: 2000 rpm; Ultrasonic Duration: 1 sec, Ultrasonic Level: 100%, Tray Type: General Purpose; Background Time: 7500 msec, Measurement Time: 7500 msec; Obscuration Limits: 10.0 - 20.0 %; Auswertung. Fraunhofer; Ausführung gemäß ISO 13320-1 sowie den Angaben des technischen Handbuchs und den Spezifikationen des Geräteherstellers
10. Bestimmung der BET-Oberfläche und des BET-Porenvolumens (Single point adsorption total pore volume):
   Durchführung und Auswertung gemäß Literatur "BET Surface Area by Nitrogen Adsorption", S.Brunauer et al. (Journal of American Chemical Society, 60, 9, 1938), der Vorgaben des Geräteherstellers sowie der DIN ISO 9277; Gerät: ASAP 2420 V 1.03a Z der Fa.Micromeritics Instrument Corporation (USA); Stickstoff; volumetrisches Verfahren; Einwaage ca. 3g +/-10%, mit Probenvorbereitung (Ausheizung 3,0°C/min. bis Zieltemperatur 50°C: 5 Stunden/50°C)
11. Gehaltsbestimmung der Carbonat- und Magnesium-haltigen Ansätze über eine Dichtebestimmung mittels Densimeter mit Schwingungswandler: Dichtemessgerät Anton Paar DMA 4500 (Österreich); Messung bei 70°C;
   Durchführung angelehnt an Bestimmung: "Relative Dichte, Ph.Eur. 6. Ausgabe, Grundwerk 2008, Band 1, Seite 33, Methode 2.2.5 und Europäische Norm EN ISO 15212-1: 1999 Deutsche Fassung: Dichtemessgeräte nach dem Schwingerprinzip
12. Gehaltsbestimmung Magnesium (berechnet als MgO): gemäß Ph.Eur. 6. Ausgabe "Schweres, basisches Magnesiumcarbonat"

Die in der vorhergehenden Beschreibung gemachten Bezugnahmen auf allgemeingültige Reinheitsvorschriften für Magnesiumhydroxidcarbonat der Qualität "schwer" beziehen sich auf die im Folgenden genannten Reviews, Monographien bzw. Richtlinien:
**1. PhEur:** "Schweres, basisches Magnesiumcarbonat" bzw. "Magnesii subcarbonas ponderosus" Europäisches Arzneibuch 6.Ausgabe, Grundwerk 2008, Band 3, Monograpien K-Z, Amtliche deutsche Ausgabe, Seite 3143-3144 Deutscher Apotheker Verlag Stuttgart, Govi-Verlag - Pharmazeutischer Verlag GmbH Eschborn ISBN 978-3-7692-3962-1
**2. BP:** "Heavy Magnesium Carbonate", British Pharmacopoeia (BP) 2009, Volume II, General Notices Monographs, Medicinal and Pharmaceutical Substances (J-Z), ISBN 978 0 11 322799 0, Seite 1263-1264
**3. USP:** "Magnesium Carbonate", The United States Pharmacopeia 2009, USP 32 - NF 27, Volume 3, USP Monographs M-Z, ISBN 1-889788-69-2, USP 32, Seite 2828-2829
**4. E 504:** " E 504 (ii) Magnesiumhydroxidcarbonat", Richtlinie 2008/84/EG Der Kommission vom 27.August 2008 zur Festlegung spezifischer Reinheitskriterien für andere Lebensmittelzusatzstoffe als Farbstoffe und Süßungsmittel, Amtsblatt der Europäischen Union vom 20.09.2008 DE, Seite L 253/119

### Liste der Tabellen und Abbildungen:

Tabelle 1: Reaktionsbedingungen, Gehalt (der erfindungsgemäßen Beispiele A - E)
Tabellen 2 und 3: DC- Magnesiumhydroxidcarbonat, schwer: galenische Eigenschaften, Schüttdichte, Stampfdichte, Fließwinkel, Kornverteilung, BET-Oberfläche, Porenvolumen (erfindungsgemäße Beispiele A - E im Vergleich zu Pulver F und im Vergleich zu DC-Magnesiumcarbonaten des Marktes ohne Bindemittel G, H, sowie mit Bindemittel I - L)
Tabelle 4: DC- Magnesiumhydroxidcarbonat, schwer: Tablettierungsdaten (erfindungsgemäße Beispiele A-E im Vergleich zu Pulver F und im Vergleich zu DC-Magnesiumcarbonaten des Marktes ohne Bindemittel G,H)
Tabelle 5: DC- Magnesiumhydroxidcarbonat, schwer: Tablettierungsdaten (erfindungsgemäße Beispiele A-E im Vergleich zu Pulver F und im Vergleich zu DC-Magnesiumcarbonate des Marktes mit 10% Stärke I-L)

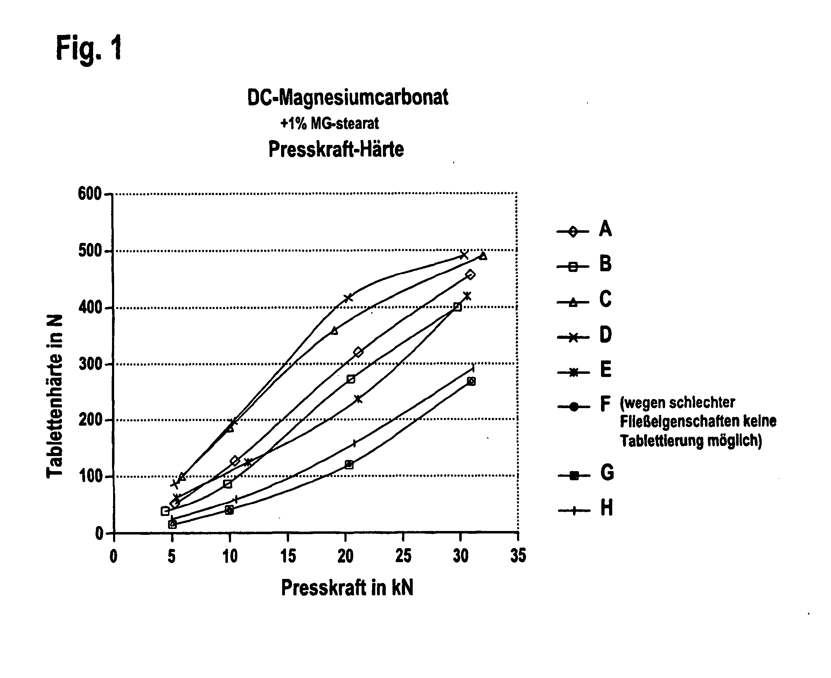

Abb. 1: DC- Magnesiumhydroxidcarbonat, schwer: Tablettierungsdaten (erfindungsgemäße Beispiele A-E vs. Pulver F vs. DC-Magnesiumcarbonate des Marktes ohne Bindemittel G,H), Tablettenhärten in Abhängigkeit von der Preßkraft, Daten der Tabelle 4

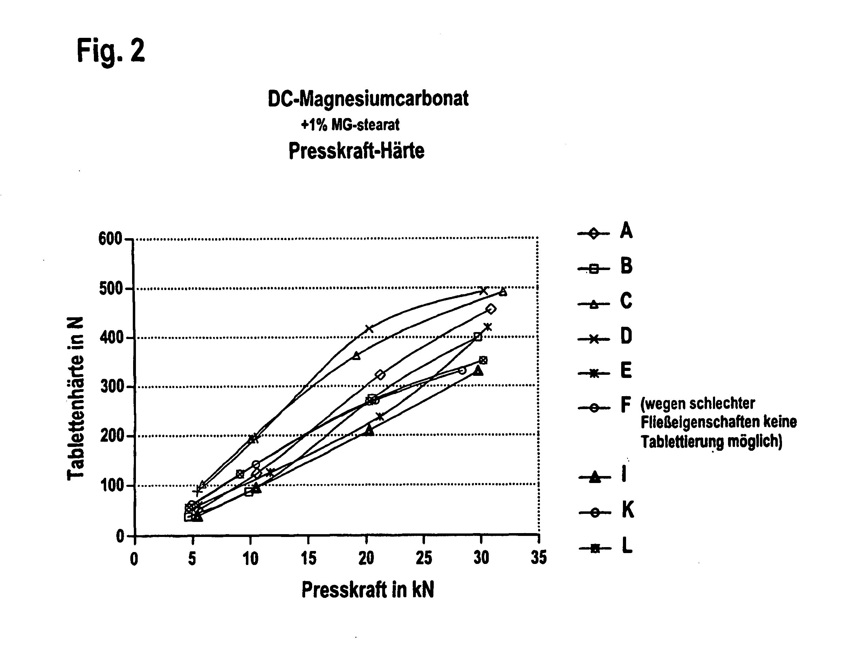

Abb. 2: DC- Magnesiumhydroxidcarbonat, schwer: Tablettierungsdaten (erfindungsgemäße Beispiele A-E im Vergleich zu Pulver F und im Vergleich zu DC-Magnesiumcarbonaten des Marktes mit 10% Stärke I-L), Tablettenhärten in Abhängigkeit von der Preßkraft; Daten der Tabelle 5

In der weiteren Beschreibung werden zum besseren Verständnis und zur Verdeutlichung der Erfindung Beispiele für das erfindungsgemäße Verfahren zur Herstellung des erfindungsgemäßen Magnesiumhydroxidcarbonats gegeben, die im Rahmen des Schutzbereichs der vorliegenden Erfindung liegen. Diese Beispiele dienen auch zur Veranschaulichung möglicher Verfahrensvarianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Die in den Beispielen und der Beschreibung sowie in den Ansprüchen gegebenen Temperaturen gelten immer in °C. Wo nicht anders bezeichnet sind Gehaltsangaben als Gew.-% bzw. Gewichtsverhältnisse aufgeführt. Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gewichts-, mol- oder Volumen-% bezogen auf die Gesamtzusammensetzung aufaddieren und nicht darüber hinausgehen können, auch wenn sich aus den angegebenen Prozentbereichen in Tabellen höhere Werte ergeben könnten. Sofern nichts anderes angegeben ist, gelten %-Angaben als Gew.-%, mit Ausnahme von Verhältnissen, die in Volumenangaben wiedergegeben sind.

### Beispiele

### Herstellung von Magnesiumhydroxidcarbonat

Die Herstellung von Magnesiumhydroxidcarbonat in beschriebener Qualität wird durch eine kontinuierliche Fällung des Produktes aus Natriumcarbonat-Lösung und Magnesiumchloridlösung, Abtrennung der Reaktionsprodukte und Trocknung erreicht:
Aus Natriumcarbonat, Na₂CO₃ wird unter Erwärmen eine Lösung hergestellt. Die Herstellung der Magnesiumchloridlösung verläuft als stark exotherme Reaktion bei Verwendung von Magnesiumchlorid- Anhydrat, MgCl₂. Der pH-Wert wird durch geringe Zugabe von Salzsäure 37% oder Magnesiumoxid auf pH 5-5,5 eingestellt.

Für die kontinuierliche Präparation werden eine heiße 3 - 15 %ige Carbonat-Lösung und eine heiße 2 - 10 %ige Mg- Lösung in einen Rohrreaktor mittels Pumpen eingeführt. (Die Lösungen werden so angesetzt, dass jeweils in den Lösungen die angegebenen Gew.-% Carbonat-, bzw. Magnesiumionen enthalten sind bezogen auf das Gesamtgewicht der Lösungen.) Die Komponenten werden im molaren Verhältnis 0,6 - 0,8 mol Magnesiumionen zu 1 mol Carbonationen zur Reaktion gebracht. Die Gehaltseinstellungen der Lösungen erfolgen praktischerweise über eine Korrelation zur Dichte der Lösungen.

Der verwendete Reaktor hat die Dimension von 300 mm Innendurchmesser zu 3300 mm Länge. Für die Reaktion wurde eine mittlere Verweilzeit im Reaktor von 7 bis 15 min bestimmt.

Die Fällung erfolgt sofort unter Beachtung des pH-Wertes (pH 8,5-9,0) und der Temperatur von 60 - 70°C ohne zusätzliche Wärmequelle. Die entstandene Suspension wird für einen gleichmäßigen Produktzulauf zur Filtrationsanlage und zur Vervollständigung der Reaktion in einem Behälter zwischengelagert, bevor sie auf einer Bandfilteranlage abgetrennt und entsprechend der spezifizierten chemischen Qualitätsparameter gewaschen wird.

Der dort anfallende Filterkuchen wird mit heißer Luft bei < 250°C, vorzugsweise bei 70 -140°C getrocknet um das Produktes entsprechend der Zusammensetzung 4MgCO₃•Mg(OH)₂•4H₂O zu erhalten.

Auf diese Weise wird das gemäß Ph.Eur., USP, BP, E 504 als "Magnesiumcarbonat basisch schwer" charakterisierte Magnesiumhydroxidcarbonat erhalten.

Im folgenden werden in der Tabelle1 zum Vergleich Daten verschiedener Versuchsläufe wiedergegeben, in denen jeweils der Salzgehalt kontrolliert eingestellt wurde.

**Tabelle 1: Reaktionsbedingungen, Gehalte, erfindungsgemäße Beispiele A - E**

| | Beispiel A | Beispiel B | Beispiel C | Beispiel D | Beispiel E |
|---|---|---|---|---|---|
| Gehalt Carbonat [%] | 15,3 | 3,0 | 7,3 | 9,6 | 6,9 |
| Gehalt Magnesium [%] | 2,4 | 3,8 | 3,6 | 4,1 | 9,7 |
| Temperatur Carbonatlösung[°C] | 82 | 80 | 80 | 80 | 80 |
| Temperatur Magnesiumsalzlösung [°C] | 70 | 71 | 70 | 70 | 70 |
| pH-Wert Magnesiumsatzlösung | 5,0 | 4,7 | 5,2 | 5,2 | 4,9 |
| Temperatur Fällung [°C] | 68 | 65 | 67 | 61 | 66 |
| pH- Wert Fällung | 9 | 9 | 9 | 9 | 9 |
| Trockenverlust nach Filtration [%] | 48 | 53 | 50 | 57 | 54 |
| Temperatur (Zuluft) Trocknung [°C] | 130 | 130 | 70 | 90 | 130 |

### Beschreibung der Trocknung für Beispiel C:

2000 g des vom Bandfilter entnommenen feuchten Materials (50-60% anhaftende Wasseranteile) werden in einem Wirbelbettgerät GPCG 5/Fa.Glatt (Deutschland) vorgelegt. Das Material wird mit Warmluft (Zulufttemperatur 70° bis 70,6°C; Ablufttemperatur 38,6° bis 42,7°C; bei einer Luftmenge von 396 bis 416 Nm³/h)) durchströmt bis es sich in seine Feinanteile zerlegt - evtl. hartnäckige Verklumpungen werden durch die Siebung über ein 710µm Sieb beseitigt. Beginnt das Material nach ca. 20 Minuten Trocknung zu fluidisieren wird die Abluftmenge auf 140 bis 160 Nm³/h reduziert (die Zuluft- und Ablufttemparaturen bleiben unverändert) und nochmals 30 Minuten unter diesen Bedingungen getrocknet. Nach insgesamt 50 - 55 Minuten Trocknungszeit (und einer rel. Feuchte von 20-21% in der Abluft bei 36°C) wird der Prozess beendet und eine Gehaltsbestimmung gem. Ph. Eur. durchgeführt. Praktische Ausbeute 780 g (geringe Materialverluste in der Anlage z.B. in den Filtern und Anhaftungen an den Anlagewänden). Sollte die Gehaltsbestimmung (berechnet als MgO) einen Mindergehalt anzeigen so muss nachgetrocknet werden.

### Beschreibung der Trocknung für Beispiel D:

2000 g des vom Bandfilter entnommenen feuchten Materials (50-60% anhaftende Wasseranteile) werden in einem Wirbelbettgerät GPCG 5/ Fa.Glatt (Deutschland) vorgelegt. Das Material wird mit Warmluft (Zulufttemperatur 87,3° bis 90,3°C; Ablufttemperatur 39,5° bis 48,2°C; bei einer Luftmenge von 399 bis 427 Nm³/h)) durchströmt bis es sich in seine Feinanteile zerlegt - evtl. hartnäckige Verklumpungen werden durch die Siebung über ein 710µm Sieb beseitigt. Beginnt das Material nach ca. 20 Minuten Trocknung zu fluidisieren wird die Abluftmenge auf 142 bis 159 Nm³/h reduziert (die Zuluft- und Ablufttemparaturen bleiben unverändert) und nochmals 30 Minuten unter diesen Bedingungen getrocknet. Nach insgesamt 50 - 55 Minuten Trocknungszeit (und einer rel. Feuchte von 15-16% in der Abluft bei 40°C) wird der Prozess beendet und eine Gehaltsbestimmung gem. Ph.Eur. durchgeführt. Praktische Ausbeute 780g (geringe Materialverluste in der Anlage z.B. in den Filtern und Anhaftungen an den Anlagewänden). Sollte die Gehaltsbestimmung (berechnet als MgO) einen Mindergehalt anzeigen, so muß nachgetrocknet werden.

**Tabelle 2:**

| | A | B | C | D | E | F |
|---|---|---|---|---|---|---|
| Schüttdichte [g/ml] | 0,43 | 0,55 | 0,42 | 0,42 | 0,53 | 0,49 |
| Stampfdichte [g/ml] | 0,57 | 0,73 | 0,59 | 0,58 | 0,75 | 0,70 |
| Fließwinkel [°] | 42,1 | 41,0 | 40,2 | 39,8 | 45,0 | 47,4 |
| Kornverteilung [Vol.%] | | | | | | |
| D (0,10) | 10,8 | 8,7 | 9,7 | 9,8 | 6,4 | 3,8 |
| D (0,25) | 34,4 | 21,2 | 19,1 | 19,6 | 15,0 | 15,8 |
| D (0,50) | 59,6 | 34,1 | 31,5 | 32,4 | 24,6 | 36,1 |
| D (0,75) | 88,4 | 49,7 | 48,0 | 49,2 | 36,8 | 60,5 |
| D (0,90) | 118,1 | 66,0 | 66,5 | 67,7 | 50,3 | 85,4 |
| BET-Oberfläche [m²/g] | 50 | 60 | 67 | 70 | 44 | 16 |
| BET-Porenvolumen [cm³/g] | 0,28 | 0,29 | 0,28 | 0,30 | 0,20 | 0,08 |

**Tabelle 3:**

| | G | H | I | K | L |
|---|---|---|---|---|---|
| Schüttdichte [g/ml] | 0,63 | 0,59 | 0,45 | 0,39 | 0,53 |
| Stampfdichte [g/ml] | 0,77 | 0,74 | 0,55 | 0,47 | 0,68 |
| Fließwinkel [°] | 33,8 | 35,1 | 30,3 | 28,4 | 39,9 |
| Kornverteilung [Vol.%] | | | | | |
| D (0,10) | 2,1 | 1,8 | 14,3 | 5,8 | 6,8 |
| D (0,25) | 6,0 | 3,3 | 29,3 | 17,1 | 19,6 |
| D (0,50) | 16,9 | 9,6 | 45,0 | 37,2 | 39,2 |
| D (0,75) | 34,3 | 46,0 | 63,3 | 59,9 | 61,5 |
| D (0,90) | 65,0 | 99,1 | 81,8 | 82,8 | 84,0 |
| BET-Oberfläche [m²/g] | 12 | 32 | 15 | 6 | 6 |
| BET-Porenvolumen [cm³/g] | 0,09 | 0,21 | 0,09 | 0,03 | 0,04 |

**Tabelle 4:**

| Muster | Preßkraft [kN] | | Tablettenhärte n. 1 Tag [N] | Friabilität [%] |
|---|---|---|---|---|
| | Soll | Ist | | |
| **A** | 5 | 5,4 | 52,0 | 0,92 |
| | 10 | 10,6 | 124,6 | 0,14 |
| | 20 | 21,3 | 321,5 | 0,06 |
| | 30 | 31,0 | 457,0 | 0,01 |
| **B** | 5 | 4,6 | 37,6 | 1,11 |
| | 10 | 9,9 | 88,3 | 0,05 |
| | 20 | 20,6 | 272,9 | 0 |
| | 30 | 29,8 | 397,1 | 0 |
| **C** | 5 | 5,9 | 103,8 | 0,01 |
| | 10 | 10,1 | 189,3 | 0,01 |
| | 20 | 19,2 | 361,2 | 0 |
| | 30 | 32,1 | 491,6 | 0 |
| **D** | 5 | 5,4 | 88,1 | 0,11 |
| | 10 | 10,5 | 194,7 | 0 |
| | 20 | 20,4 | 415,9 | 0 |
| | 30 | 30,4 | 492,2 | 0 |
| **E** | 5 | 5,5 | 61,0 | 0,66 |
| | 10 | 11,7 | 124,5 | 0,07 |
| | 20 | 21,2 | 238,0 | 0 |
| | 30 | 30,7 | 419,1 | 0 |
| **F** | 5 | Wegen | schlechter Fließeigenschaften | keine Tablettierung möqlich! |
| | 10 | | | |
| | 20 | | | |
| | 30 | | | |
| **G** | 5 | 5,2 | 15,1 | 6,92 |
| | 10 | 10,1 | 39,9 | 1,20 |
| | 20 | 20,4 | 120,2 | 0,22 |
| | 30 | 31,0 | 269,9 | 0,11 |
| **H** | 5 | 5,0 | 24,2 | 3,70 |
| | 10 | 10,5 | 59,1 | 0,34 |
| | 20 | 20,8 | 158,6 | 0,08 |
| | 30 | 31,0 | 290,5 | 0,04 |

**Tabelle 5:**

| Muster | Preßkraft [kN] | | Tablettenhärte n. 1 Tag [N] | Friabilität [%] |
|---|---|---|---|---|
| | Soll | Ist | | |
| **A** | 5 | 5,4 | 52,0 | 0,92 |
| | 10 | 10,6 | 124,6 | 0,14 |
| | 20 | 21,3 | 321,5 | 0,06 |
| | 30 | 31,0 | 457,0 | 0,01 |
| **B** | 5 | 4,6 | 37,6 | 1,11 |
| | 10 | 9,9 | 88,3 | 0,05 |
| | 20 | 20,6 | 272,9 | 0 |
| | 30 | 29,8 | 397,1 | 0 |
| **C** | 5 | 5,9 | 103,8 | 0,01 |
| | 10 | 10,1 | 189,3 | 0,01 |
| | 20 | 19,2 | 361,2 | 0 |
| | 30 | 32,1 | 491,6 | 0 |
| **D** | 5 | 5,4 | 88,1 | 0,11 |
| | 10 | 10,5 | 194,7 | 0 |
| | 20 | 20,4 | 415,9 | 0 |
| | 30 | 30,4 | 492,2 | 0 |
| **E** | 5 | 5,5 | 61,0 | 0,66 |
| | 10 | 11,7 | 124,5 | 0,07 |
| | 20 | 21,2 | 238,0 | 0 |
| | 30 | 30,7 | 419,1 | 0 |
| **F** | 5 | Wegen | schlechter Fließeigenschaften | keine Tablettierung möglichl |
| | 10 | | | |
| | 20 | | | |
| | 30 | | | |
| **I** | 5 | 5,4 | 39,4 | 0,47 |
| | 10 | 10,5 | 92,0 | 0,07 |
| | 20 | 20,3 | 207,4 | 0,01 |
| | 30 | 29,8 | 329,5 | 0,08 |
| **K** | 5 | 4,8 | 58,9 | 0,16 |
| | 10 | 10,4 | 139,5 | 0,09 |
| | 20 | 20,7 | 268,3 | 0,06 |
| | 30 | 28,3 | 329,3 | 0,08 |
| **L** | 5 | 4,7 | 56,5 | 0,18 |
| | 10 | 9,2 | 121,5 | 0,11 |
| | 20 | 20,4 | 267,5 | 0,08 |
| | 30 | 30,4 | 350,8 | 0,05 |

### Zu Vergleichsversuchen eingesetzte, im Handel erhältliche Qualitäten von Pulverware sowie von direkt verpreßbaren Magnesiumhydroxidcarbonaten, schwer:

**F:** Magnesiumhydroxidcarbonat, schwer, reinst, Ph EUR, BP, USP, E 504, Merck KGaA, Darmstadt [Deutschland], Art. Nr. 1.05829, Charge K38796529 (es handelt sich um eine Pulverware ohne ausgelobte DC-Eigenschaften)
**G:** NutriMag MC DC Magnesiumcarbonat schwer, pharmazeutische Qualität, granuliert, in der Reinheit gemäß BP, USP, Ph.Eur, CALMAGS GmbH, Lüneburg [Deutschland], Charge: 308075060
**H:** Pharmagnesia MC Type A Granulat, Magnesiumcarbonat, schwer, Granulat, pharmazeutisch, EP, E504, Lehmann & Voss, Hamburg [Deutschland], Art. Nr. 2420230, Charge: 0805-089
**I:** SCORAMAG DC 90ST mit 10% Stärke, Scora, Caffiers [Frankreich], Charge: 07/348/C414
**K:** Magnesiumcarbonat DC 90S/C, Granulat, mit ca. 10% Maisstärke, Dr. Paul Lohmann, Emmerthal [Deutschland], Art. Nr. 501003036270, Charge: 234298
**L:** Magnesiumcarbonat DC 90S/F, Granulat, mit ca. 10% Maisstärke, Dr. Paul Lohmann, Emmerthal [Deutschland], Art. Nr. 501003036280, Charge: 138252

## Patentansprüche

1. Direkt verpreßbares Magnesiumhydroxidcarbonat, (schwer gemäß den Anforderungen der PhEur, BP, USP und E 504), **dadurch gekennzeichnet, dass** es eine BET-Oberfäche von mindestens 44 bis 70 m²/g, eine Schüttdichte von 0,40 bis 0,60 g/ml und eine Stampfdichte von 0,50 bis 0,80 g/ml aufweist.

2. Direkt verpreßbares Magnesiumhydroxidcarbonat gemäß Anspruch 1, **dadurch gekennzeichnet dass** es einen mittleren Partikeldurchmesser (Laser; D_{0,50}) im Bereich zwischen 20 und 60 µm, insbesondere im Bereich von 24 bis 60 µm aufweist.

3. Direkt verpreßbares Magnesiumhydroxidcarbonat gemäß einem der Ansprüche 1 oder 2 , **dadurch gekennzeichnet, dass** es eine BET-Oberfäche von größer 50 m²/g aufweist.

4. Direkt verpreßbares Magnesiumhydroxidcarbonat gemäß einem oder mehreren der Ansprüche 1 bis 3, erhältlich durch kontinuierliche Reaktion in einem Rohrreaktor, indem aus einer erwärmten Lösung, die durch Vermischen einer Lösung eines Magnesiumsalzes mit einem Magnesiumgehalt in der Lösung von 2 - 11 Gew.-% und einer Lösung eines Alkali- oder Erdalkalicarbonats mit einem Carbonatgehalt in der Lösung von 2 - 18 Gew.-% erhalten wird, bei einer Temperatur im Bereich von 60 bis 70°C bei einem pH-Wert von 8,5 - 9,0 Magnesiumcarbonat ausgefällt und abfiltriert wird und anschließend in einem Konvektionstrockner einer Trocknung bis auf einen Gehalt an Magnesiumhydroxidcarbonat von 40 bis 43,5 Gew.-%, berechnet als MgO, unterzogen wird.

5. Direkt verpreßbares Magnesiumhydroxidcarbonat gemäß Anspruch 4, erhältlich durch kontinuierliche Reaktion in einem Rohrreaktor, wobei aus einer erwärmten Lösung ausgefällt wird, die durch Vermischen einer Lösung mit einem Magnesiumgehalt in der Lösung von 3 - 6 Gew.-% und einer Lösung mit einem Carbonatgehalt in der Lösung von 2 - 18 Gew.-% erhalten wird.

6. Tabletten, welche Härten im Bereich >80 N bis >200 N und eine Friabilität < 0,2 Gew.-% aufweisen und welche hergestellt sind unter Verwendung eines Magnesiumhydroxidcarbonats der Ansprüche 1 - 3 durch Kompression mit einer Preßkraft im Bereich von 10 kN bis 20 kN.

7. Tabletten gemäß Anspruch 6, aufweisend eine Friabilität <0,1 Gew.-%.

8. Verfahren zur Herstellung eines direkt verpressbaren Magnesiumhydroxidcarbonats gemäß einem oder mehreren der Ansprüche 1 - 5, **dadurch gekennzeichnet, dass**
a) eine erwärmte Lösung eines Magnesiumsalzes und eine erwärmte Lösung eines Alkali-oder Erdalkalicarbonats kontinuierlich in einen Rohrreaktor gepumpt und miteinander vermischt werden, wobei die Lösung des Magnesiumsalzes einen Magnesiumgehalt von 2 - 11 Gew.-% und die Lösung des Alkali-oder Erdalkalicarbonats einen Carbonatgehalt 2 - 18 Gew.-% aufweist, so dass sich in der Reaktionslösung eine Temperatur von 60 bis 70°C und ein pH-Wert im Bereich von 8,5 - 9,0 einstellen und das gebildete Magnesiumhydroxidcarbonat ausgefällt wird
und
b) das aus der produkthaltigen Reaktionsmischung ausgefällte Produkt, gegebenenfalls nach einiger Zeit ruhen lassen, abfiltriert und getrocknet wird.

9. Verfahren gemäß Anspruch 8, **dadurch gekennzeichnet, dass** eine erwärmte Lösung eines Magnesiumsalzes mit einem Magnesiumgehalt von 3 - 6 Gew.-% und eine Lösung eines Alkali-oder Erdalkalicarbonats mit einem Carbonatgehalt von 3 - 15 Gew.-% kontinuierlich in einen Rohrreaktor gepumpt und vermischt werden.

10. Verfahren gemäß Anspruch 8 oder 9, **dadurch gekennzeichnet, dass** das abfiltrierte Produkt in einem Wirbelschichttrockner oder in einem Konvektionstrockner getrocknet wird.

11. Verfahren gemäß der Ansprüche 8 oder 9 und 10, **dadurch gekennzeichnet, dass** das abfiltrierte Produkt auf ein Gehalt von 40 bis 43,5 Gew.-% an Magnesiumcarbonat (berechnet als MgO) getrocknet wird.

12. Verwendung eines direkt verpreßbaren Magnesiumhydroxidcarbonats gemäß einem oder mehreren der Ansprüche 1 bis 5 zur Herstellung von Tabletten mit einer Härte >80 N und einer Friabilität < 0,2 Gew. % durch Verpressen mit einer Presskraft von10kN bis 20 kN.

13. Verwendung eines direkt verpreßbaren Magnesiumhydroxidcarbonats gemäß einem oder mehreren der Ansprüche 1 bis 5 zur Herstellung von Tabletten mit einer Härte >80 N und einer Friabilität < 0,1Gew. % durch Verpressen mit einer Presskraft von 10kN bis 20 kN.

14. Verwendung eines direkt verpreßbaren Magnesiumhydroxidcarbonats gemäß einem oder mehreren der Ansprüche 1 bis 5 als Bestandteil in wirkstoffhaltigen Tablettenformulierungen, Kau- und Lutschtabletten, Brausetabletten Brausepulvern, in Kapselformulierungen oder in Pulverzubereitungen zur Magnesiumanreicherung.

15. Verwendung eines direkt verpreßbaren Magnesiumhydroxidcarbonats gemäß einem oder mehreren der Ansprüche 1 bis 5, zur Herstellung von Tablettenformulierungen, enthaltend Vitamine, Mineralstoffe, Spurenelemente, funktionelle Lebensmittelbestandteile oder Wirkstoffe.

16. Verwendung eines direkt verpreßbaren Magnesiumhydroxidcarbonats gemäß einem oder mehreren der Ansprüche 1 bis 5 zur Herstellung von Tablettenformulierungen, enthaltend synthetische oder natürliche Farbstoffe, natürliche und/oder naturidentischen Aromastoffen und/oder andere geschmacksgebende Substanzen, wie z.B. aus der Gruppe Aspartam, Saccharin, Acesulfam K, Neohesperidin, Sucralose, Thaumatin und Steviosid, oder Fruchtaromen, Fruchtsäuren, geschmacksgebende Pflanzenauszüge und pharmazeutische oder diätetische Wirkstoffe.

## Claims

1. Directly compressible magnesium hydroxide carbonate, (heavy in accordance with the requirements of PhEur, BP, USP and E 504), **characterised in that** it has a BET surface area of at least 44 to 70 m²/g, a bulk density of 0.40 to 0.60 g/ml and a tapped density of 0.50 to 0.80 g/ml.

2. Directly compressible magnesium hydroxide carbonate according to Claim 1, **characterised in that** it has an average particle diameter (laser; D_{0.50}) in the range between 20 and 60 µm, in particular in the range from 24 to 60 µm.

3. Directly compressible magnesium hydroxide carbonate according to one of Claims 1 or 2, **characterised in that** it has a BET surface area of greater than 50 m²/g.

4. Directly compressible magnesium hydroxide carbonate according to one or more of Claims 1 to 3, obtainable by continuous reaction in a tubular reactor by precipitating magnesium carbonate out from a warmed solution obtained by mixing a solution of a magnesium salt having a magnesium content in the solution of 2 - 11 % by weight and a solution of an alkali-metal or alkaline-earth metal carbonate having a carbonate content in the solution of 2 - 18% by weight at a temperature in the range from 60 to 70°C at a pH of 8.5 - 9.0, filtering off the magnesium carbonate and subsequently drying it in a convection dryer to a magnesium hydroxide carbonate content of 40 to 43.5% by weight, calculated as MgO.

5. Directly compressible magnesium hydroxide carbonate according to Claim 4, obtainable by continuous reaction in a tubular reactor, where the precipitation is carried out from a warmed solution obtained by mixing a solution having a magnesium content in the solution of 3 - 6% by weight and a solution having a carbonate content in the solution of 2 - 18% by weight.

6. Tablets which have hardnesses in the range >80 N to >200 N and a friability < 0.2% by weight and which have been produced using a magnesium hydroxide carbonate of Claims 1 - 3 by compression with a pressing force in the range from 10 kN to 20 kN.

7. Tablets according to Claim 6, having a friability <0.1 % by weight.

8. Process for the preparation of a directly compressible magnesium hydroxide carbonate according to one or more of Claims 1 - 5, **characterised in that**
a) a warmed solution of a magnesium salt and a warmed solution of an alkali-metal or alkaline-earth metal carbonate are pumped continuously into a tubular reactor and mixed with one another, where the solution of the magnesium salt has a magnesium content of 2 - 11 % by weight and the solution of the alkali-metal or alkaline-earth metal carbonate has a carbonate content of 2 -18% by weight, so that a temperature of 60 to 70°C and a pH in the range 8.5 - 9.0 become established in the reaction solution and the magnesium hydroxide carbonate formed is precipitated out
and
b) the product precipitated out from the product-containing reaction mixture is optionally allowed to rest after some time, filtered off and dried.

9. Process according to Claim 8, **characterised in that** a warmed solution of a magnesium salt having a magnesium content of 3 - 6% by weight and a solution of an alkali-metal or alkaline-earth metal carbonate having a carbonate content of 3 - 15% by weight are pumped continuously into a tubular reactor and mixed.

10. Process according to Claim 8 or 9, **characterised in that** the filtered-off product is dried in a fluidised-bed dryer or in a convection dryer.

11. Process according to Claim 8 or 9 or 10, **characterised in that** the filtered-off product is dried to a content of 40 to 43.5% by weight of magnesium carbonate (calculated as MgO).

12. Use of a directly compressible magnesium hydroxide carbonate according to one or more of Claims 1 to 5 for the production of tablets having a hardness >80 N and a friability < 0.2% by weight by compression with a pressing force of 10 kN to 20 kN.

13. Use of a directly compressible magnesium hydroxide carbonate according to one or more of Claims 1 to 5 for the production of tablets having a hardness >80 N and a friability < 0.1 % by weight by compression with a pressing force of 10 kN to 20 kN.

14. Use of a directly compressible magnesium hydroxide carbonate according to one or more of Claims 1 to 5 as constituent in active compound-containing tablet formulations, chewable tablets and lozenges, effervescent tablets, effervescent powders, in capsule formulations or in powder preparations for magnesium enrichment.

15. Use of a directly compressible magnesium hydroxide carbonate according to one or more of Claims 1 to 5 for the preparation of tablet formulations comprising vitamins, mineral substances, trace elements, functional food constituents or active compounds.

16. Use of a directly compressible magnesium hydroxide carbonate according to one or more of Claims 1 to 5 for the preparation of tablet formulations comprising synthetic or natural dyes, natural and/or nature-identical aromas and/or other flavouring substances, such as, for example, from the group aspartame, saccharin, acesulfame K, neohesperidine, sucralose, thaumatin and stevioside, or fruit aromas, fruit acids, flavouring plant extracts and pharmaceutical or dietetic active compounds.

## Revendications

1. Carbonate d'hydroxyde de magnésium directement compressible (lourd, conformément aux exigences de PhEur, BP, USP et E 504), **caractérisé en ce qu'**il présente une aire de surface BET d'au moins 44 à 70 m²/g, une densité dans la masse de 0,40 à 0,60 g/ml et une densité compactée de 0,50 à 0,80 g/ml.

2. Carbonate d'hydroxyde de magnésium directement compressible selon la revendication 1, **caractérisé en ce qu'**il présente un diamètre de particule moyen (laser ; D_{0,50}) dans la plage entre 20 et 60 µm, en particulier dans la plage de 24 à 60 µm.

3. Carbonate d'hydroxyde de magnésium directement compressible selon l'une des revendications 1 ou 2, **caractérisé en ce qu'**il présente une aire de surface BET supérieure à 50 m²/g.

4. Carbonate d'hydroxyde de magnésium directement compressible selon une ou plusieurs des revendications 1 à 3, pouvant être obtenu par réaction continue dans un réacteur tubulaire en précipitant du carbonate de magnésium à partir d'une solution chauffée obtenue en mélangeant une solution d'un sel de magnésium présentant une teneur en magnésium dans la solution de 2 - 11 % en poids et une solution d'un carbonate de métal alcalin ou de métal alcalino-terreux présentant une teneur en carbonate dans la solution de 2 - 18% en poids à une température dans la plage de 60 à 70°C à un pH de 8,5 - 9,0, en récupérant par filtration le carbonate de magnésium et en le séchant ensuite dans un séchoir à convexion jusqu'à une teneur en carbonate d'hydroxyde de magnésium de 40 à 43,5% en poids, calculée en tant que MgO.

5. Carbonate d'hydroxyde de magnésium directement compressible selon la revendication 4, pouvant être obtenu par réaction continue dans un réacteur tubulaire, où la précipitation est mise en oeuvre à partir d'une solution chauffée obtenue en mélangeant une solution présentant une teneur en magnésium dans la solution de 3 - 6% en poids et une solution présentant une teneur en carbonate dans la solution de 2 - 18% en poids.

6. Comprimés présentant des duretés dans la plage >80 N à >200 N et une friabilité < 0,2% en poids et produits en utilisant un carbonate d'hydroxyde de magnésium selon les revendications 1 - 3 par compression selon une force de pression dans la plage de 10 kN à 20 kN.

7. Comprimés selon la revendication 6, présentant une friabilité <0, 1 % en poids.

8. Procédé pour la préparation d'un carbonate d'hydroxyde de magnésium directement compressible selon une ou plusieurs des revendications 1 - 5, **caractérisé en ce que**
a) une solution chauffée d'un sel de magnésium et une solution chauffée d'un carbonate de métal alcalin ou de métal alcalino-terreux sont pompées en continu à l'intérieur d'un réacteur tubulaire et sont mélangées ensemble, où la solution du sel de magnésium présente une teneur en magnésium de 2 - 11 % en poids et la solution du carbonate de métal alcalin ou de métal alcalino-terreux présente une teneur en carbonate de 2 -18% en poids, de telle sorte qu'une température de 60 à 70°C et un pH dans la plage de 8,5 - 9,0 en viennent à s'établir dans la solution de réaction et que le carbonate d'hydroxyde de magnésium formé soit récupéré par précipitation
et
b) le produit récupéré par précipitation à partir du mélange de réaction contenant le produit est en option laissé au repos un certain temps, récupéré par filtration et séché.

9. Procédé selon la revendication 8, **caractérisé en ce qu'**une solution chauffée d'un sel de magnésium présentant une teneur en magnésium de 3 - 6% en poids et une solution d'un carbonate de métal alcalin ou de métal alcalino-terreux présentant une teneur en carbonate de 3 - 15% en poids sont pompées en continu à l'intérieur d'un réacteur tubulaire et sont mélangées.

10. Procédé selon la revendication 8 ou 9, **caractérisée en ce que** le produit récupéré par filtration est séché dans un séchoir à lit fluidisé ou dans un séchoir à convexion.

11. Procédé selon la revendication 8 ou 9 ou 10, caractérisé ce que le produit récupéré par filtration est séché jusqu'à une teneur de 40 à 43,5% en poids de carbonate de magnésium (calculée en tant que MgO).

12. Utilisation d'un carbonate d'hydroxyde de magnésium directement compressible selon une ou plusieurs des revendications 1 à 5 pour la production de comprimés présentant une dureté >80 N et une friabilité < 0,2% en poids par compression selon une force de pression de 10 kN à 20 kN.

13. Utilisation d'un carbonate d'hydroxyde de magnésium directement compressible selon une ou plusieurs des revendications 1 à 5 pour la production de comprimés présentant une dureté >80 N et une friabilité < 0, 1 % en poids par compression selon une force de pression de 10 kN à 20 kN.

14. Utilisation d'un carbonate d'hydroxyde de magnésium directement compressible selon une ou plusieurs des revendications 1 à 5 en tant que constituent dans des formulations de comprimés contenant des composés actifs, de comprimés et de pastilles mâchables, de comprimés effervescents, de poudres effervescentes, dans des formulations de gélules ou dans des préparations de poudres pour un enrichissement en magnésium.

15. Utilisation d'un carbonate d'hydroxyde de magnésium directement compressible selon une ou plusieurs des revendications 1 à 5 pour la préparation de formulations de comprimés comprenant des vitamines, des substances minérales, des oligo-éléments, des constituants alimentaires fonctionnels ou des composés actifs.

16. Utilisation d'un carbonate d'hydroxyde de magnésium directement compressible selon une ou plusieurs des revendications 1 à 5 pour la préparation de formulations de comprimés comprenant des colorants synthétiques ou naturels, des aromes naturels et/ou identiques à la nature et/ou d'autres substances aromatisantes, telles que, par exemple, prises parmi le groupe constitué par aspartame, saccharine, acésulfame K, néohespéridine, sucralose, thaumatin et stévioside, ou des aromes de fruits, des acides de fruits, des extraits de plantes aromatisantes et des composés actifs pharmaceutiques ou diététiques.
